# EUROPÄISCHE PATENTANMELDUNG

(11) **EP 1 790 324 A1**
(43) Veröffentlichungstag der Anmeldung: **30.05.2007**
(21) Anmeldenummer: 06124717.7
(22) Anmeldetag: 24.11.2006
(51) Int. Cl.: A61K 8/02, A61K 8/73, A61K 8/81, A61Q 5/12, A61Q 15/00, A61Q 17/04, A61Q 19/00, A61Q 19/10

(54) **Transparente oder transluzente Gele**

(30) Priorität: 28.11.2005 DE 102005056497
(71) Anmelder: Beiersdorf AG, 20245 Hamburg (DE)
(72) Erfinder: Bleckmann Andreas, 22926, Ahrensburg (DE); Riedel, Heidi, 22083, Hamburg (DE)

(57) **Zusammenfassung**

Kosmetische Zubereitung in Form eines transparenten bzw. transluzenten Gels, dadurch gekennzeichnet, dass sie
i. Wasser,
ii. Natriumcarboxymethylstärke und
iii. ein oder mehrere Hydrokolloide enthält.

## Beschreibung

Die vorliegende Erfindung betrifft kosmetische Zubereitungen in Form von transparenten oder transluzenten Gelen mit einer besonderen samtigen Textur.

Übliche und sich gerade in neuerer Zeit immer weiter verbreitende kosmetische Zubereitungsformen sind Gele.

Im technischen Sinn werden unter dem Begriff "Gel" relativ formbeständige, leicht verformbare disperse Systeme aus zumindest zwei Komponenten verstanden, welche in der Regel aus einem - meist festen - kolloid zerteilten Stoff aus langkettigen Molekülgruppierungen (z. B. Gelatine, Kieselsäure, Polysaccharide) als Gerüstbildner und einem flüssigen Dispersionsmittel (z. B. Wasser) bestehen. Der kolloidal zerteilte Stoff wird oft als Verdickungsmittel, Gelbildner oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können.

Als Verdickungsmittel werden hauptsächlich Acrylsäure-Polymere verwendet. Die vorhandenen freien Carboxy-Gruppen dieser Polymere werden durch Alkalien - wie z. B. Natronlauge oder Amine - in die Salzform überführt und führen dadurch infolge einer starken Vernetzung zu einer Viskositätserhöhung. Weiterhin kommen auch Cellulose-Derivate oder andere Polysaccharide, wie zum Beispiel Guar-Mehl oder Xanthan, als Gelbildner zum Einsatz. Je nach Art und Menge des Verdickungsmittels lässt sich die Konsistenz eines Geles von flüssig bis schnittfest einstellen.

Das Dispersionsmittel, welches das Netzwerk umgibt, zeichnet sich durch elektrostatische Affinität zum Verdickungsmittel aus, d. h., ein vorwiegend polares (insbesondere: hydrophiles) Verdickungsmittel geliert vorzugsweise ein polares Dispersionsmittel (insbesondere: Wasser), wohingegen ein vorwiegend unpolares Verdickungsmittel vorzugsweise unpolare Dispersionsmittel geliert.

Starke elektrostatische Wechselwirkungen, welche beispielsweise in Wasserstoffbrückenbindungen zwischen Verdickungsmittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen.

In der kosmetischen Galenik zeichnen sich Gele in aller Regel durch eine halbfeste, oft fließfähige Konsistenz aus.

Ferner sind so genannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele - fachsprachlich auch "surfactant gels" genannt - Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten.

Nachteil der Gele des Standes der Technik ist, dass sich diese bei einem höheren Gehalt an Moisturizern (z. B. größer 3 Gew.-%) nach dem Verteilen glitschig und/oder klebrig anfühlen. Zwar lässt sich die Sensorik solcher Gele durch den Einsatz bestimmter, geeigneter Hydrokolloide verbessern, allerdings muss hierbei üblicherweise in Kauf genommen werden, dass die Gele ihre Transparenz verlieren; sie werden milchig. Vollständig klare Gele mit hoher Pflegeleistung und guten sensorischen Eigenschaften sind nach dem Stand der Technik hingegen nicht erhältlich.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Während die Reinigungswirkung eines Shampoos, die Kämmbarkeitsverbesserung einer Haarspülung oder die Kräuselung des Haares durch eine Dauerwelle einfach und objektiv feststellbar sind, sind andere Wirkungen und Eigenschaften kosmetischer Produkte praktisch nicht messbar oder nur sehr individuell spürbar. Hierzu zählen zum Beispiel ein bestimmtes (belebendes, weiches, geschmeidiges, glattes etc.) Hautgefühl oder die Weichheit und Geschmeidigkeit der Haut nach der Anwendung eines Kosmetikums sowie die Fülle und Sprungkraft der Haare und dergleichen mehr. Ferner unterliegt die Erwartung der Verbraucher auch nebengeordneten Eigenschaften des Produktes. Hier sind insbesondere der Duft und die Farbe bzw. das Aussehen des Kosmetikums sowie seine Verpackung, der Preis, der Hersteller und die Werbeaussage zu nennen.

Eine für den Verbraucher sehr wesentliche, dabei aber nur schwer quantitativ messbare Eigenschaft kosmetischer Produkte ist ihre Textur. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- *Visuelle Eindrücke*: alle mit dem Auge wahrnehmbaren Merkmale (Farbe, Form, Struktur).
- *Olfaktorische Eindrücke*: alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- *Haptische Eindrücke*: alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

Die am häufigsten in der Forschung und Entwicklung genutzte Methode der sensorischen Analyse ist die Unterschiedsprüfung. Die Aufgabe beschränkt sich hierbei üblicherweise auf die Erkennung eines von mehreren Proben oder von einer Kontrollprobe abweichenden Musters. Während bei Unterschiedsprüfungen innerhalb eines Tests nur zwei Proben miteinander verglichen werden, ist bei der Rangordnungsprüfung eine Reihenfolge von drei und mehr Proben festzulegen und zwar in der Regel nach Intensität, Qualität, Beliebtheit oder Ähnlichkeit mit einer Vergleichsprobe. Diese (einfache) Methode eignet sich z. B. für eine Vorauswahl von Proben in der Produkt-Optimierung und wird auch häufig in der Marktforschung eingesetzt.

Eine Aufgabe der vorliegenden Erfindung war es, gelförmige Zubereitungen zu finden, welche neben den für Kosmetika üblichen Kriterien wie Verträglichkeit, Lagerstabilität und dergleichen auch für den Verbraucher wesentliche, bisher nicht gekannte kosmetische Leistungen bieten. Insbesondere sollten die gesuchten Zubereitungen die Nachteile des Standes der Technik nicht aufweisen, d. h. sie sollten gehaltvoll und pflegend sein und dabei gleichzeitig gute sensorische Eigenschaften aufweisen. Demensprechend sollten die Zubereitungen auch bei hohen Gehalten an Moisturizern ein angenehmes Hautgefühl erzeugen und vollständig klar sein.

Überraschend hat sich gezeigt, dass eine
kosmetische Zubereitung in Form eines transparenten bzw. transluzenten Gels, dadurch gekennzeichnet, dass sie
i. Wasser,
ii. Natriumcarboxymethylstärke und
iii. ein oder mehrere Hydrokolloide enthält
diese Aufgaben zu lösen vermag.

Die erfindungsgemäßen Zubereitungen stellen eine erhebliche Bereicherung des Standes der Technik in Bezug auf gelförmige Zubereitungen dar. Sie sind in jeglicher Hinsicht überaus befriedigende Präparate. Es war für den Fachmann nicht vorauszusehen gewesen, dass die erfindungsgemäßen Gele insbesondere sehr gehaltvoll und pflegend sein würden, wobei sie ein samtigwachsartiges Hautgefühl nach ihrer Anwendung erzeugen: Die Haut fühlt sich angenehm zart und geschmeidig an, wobei diese Wirkung den ganzen Tag anhalten kann.

Als Maß für die Pflegeleistung eines Kosmetikums kann beispielsweise die Wirkung des Produktes auf die Hautfeuchte dienen. Der Wassergehalt der Hornschicht spielt als Schutzfaktor (Barrierefunktion), aber auch beim kosmetischen Eindruck der Haut eine große Rolle. Ist er zu gering, wird die Haut trocken und rissig und kann ihre Barrierefunktion nicht mehr erfüllen. Der Grad der Befeuchtung der Hornschicht lässt sich durch Corneometrie ermitteln. Als Messsonde dient dabei ein Plattenkondensator, an dessen Rändern sich ein elektrisches Streufeld ausbildet.

Die Eigenschaften dieses elektrischen Feldes ändern sich mit dem Wassergehalt der Umgebung, durch die es verläuft. Der Plattenkondensator ist dabei durch eine hauchdünne Folie von der Haut getrennt, die verhindert, dass durch das ionenhaltige Oberflächenwasser Kurzschlüsse zwischen den Kondensatorplatten entstehen. Die Messung erfolgt in einem vollklimatisierten, konstant temperierten Raum, da das Messergebnis stark von der Aktivität der Schweißdrüsen beeinflusst werden kann.

Zur Untersuchung der Wirkung des Produktes wird die Hautfeuchtigkeit jeweils vor und zu einem definierten Zeitpunkt (beispielsweise 2 Stunden) nach einer kontrollierten Anwendung ermittelt. Es ist vorteilhaft, die Prüfmuster gegen ein unbehandeltes Kontrollareal zu prüfen.

Besonders bevorzugt stellen die erfindungsgemäßen Zubereitungen transparente Gele dar. In einer besonders bevorzugten Ausführungsform enthalten die Zubereitungen im Sinne der vorliegenden Erfindung keinerlei Ölkomponenten.

Als transparent im Sinne der vorliegenden Erfindung werden Gele bezeichnet, welche in einer Schichtdicke von 1 cm bei einer Transmissionsmessung mit einem UV/VIS-Spektrometer bei einer Wellenlänge von 600 nm eine Transmission von mehr als 90 % zeigen.

Als transluzent im Sinne der vorliegenden Erfindung werden Gele bezeichnet, welche in einer Schichtdicke von 1 cm bei einer Transmissionsmessung mit einem UV/VIS-Spektrometer bei einer Wellenlänge von 600 nm eine Transmission von mehr als 60 % zeigen.

Natriumcarboxymethylstärke mit der CAS-Nr. 9063-38-1 trägt die INCI-Bezeichnung Sodium carboxy methyl starch und ist beispielsweise unter der Handelsbezeichnung COVAGEL von der Fa. LCW erhältlich.

Es ist bevorzugt im Sinne der vorliegenden Erfindung, die Menge an Natriumcarboxymethylstärke aus dem Bereich von 0,5 bis 5 Gew.-%, besonders bevorzugt aus dem Bereich von 2 bis 3 Gew.-% - jeweils bezogen auf das Gesamtgewicht der Zubereitung - zu wählen.

Das oder die Hydrokolloide werden erfindungsgemäß vorteilhaft aus einer oder mehreren der folgenden Gruppen gewählt:
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und mikrokristalline Cellulose dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Polyurethane.

Erfindungsgemäß besonders vorteilhafte Hydrokolloide sind Verbindungen, die die INCI-Bezeichnung Ammonium Acryloyldimethyltaurate / VP Copolymer tragen.

Erfindungsgemäß vorteilhaft weisen diese Verbindungen die Summenformel [C₇H₁₆N₂SO₄]ₙ [C₆H₉NO]ₘ auf, einer statistischen Struktur wie folgt entsprechend

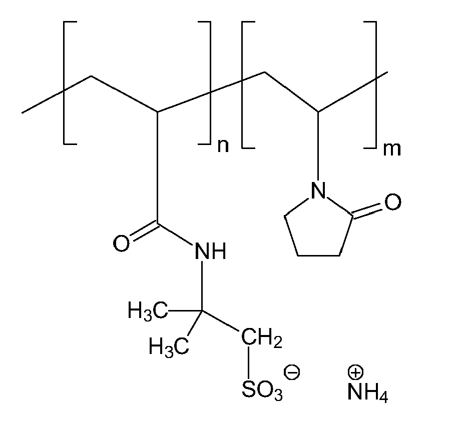

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Im folgenden werden diese Verbindungen auch als Ammoniumacryloyldimethyltaurate/Vinylpyrrolidoncopolymere bezeichnet.

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Besonders bevorzugte Hydrokolloide im Sinne der vorliegenden Erfindung sind ferner Polyacrylate, d. h. Polymere auf Basis von Estern der Acrylsäure *(Polyacrylsäureester)* der allgemeinen Strukturformel 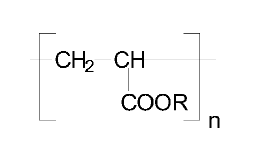 worin R für lineare, verzweigte oder cyclische, ggf. funktionelle Substituenten (z. B. Hydroxy-, Amin- oder Epoxid-Gruppen) enthaltende Alkyl-Reste steht, z. B. für Methyl-, Ethyl-, Isopropyl-, *tert-*Butyl-, Cyclohexyl-, 2-Ethylhexyl-, Dodecyl-, 2-Hydroxyethyl- und 2-Dimethylaminoethyl-.

Ganz besonders bevorzugt sind Polyacrylate mit der INCI-Bezeichnung Sodium Polyacrylate, beispielsweise das unter der Handelsbezeichnung Cosmedia SP bei der Fa. Cognis erhältliche.

Es ist bevorzugt im Sinne der vorliegenden Erfindung, das oder die Hydrokolloide aus der Gruppe Ammoniumacryloyldimethyltaurate/Vinylpyrrolidoncopolymere und Polyacrylate zu wählen.

Das Verhältnis von Natriumcarboxymethylstärke zu Hydrokolloid (insbesondere Ammoniumacryloyldimethyltaurate/Vinylpyrrolidoncopolymere und/oder Sodium Polyacrylate) wird bevorzugt wie 5 : 1 bis 1 : 1 gewählt. Besonders bevorzugt ist ein Verhältnis von ca. 2 : 1.

In einer besonders bevorzugten Ausführungsform, mit der sich die erfindungsgemäße samtige Textur besonders gut verwirklichen lässt, wobei die Formulierungen selbst besonders gehaltvoll und pflegend sind und gleichzeitig für ein samtweiches Hautgefühl nach der Anwendung sorgen, enthalten die Zubereitungen im Sinne der vorliegenden Erfindung sog. Moisturizer. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff.

Vorteilhaft wird die Menge an Moisturizern (eine oder mehrere Verbindungen) aus dem Bereich von 1 bis 20 Gew.-%, bevorzugt von 3 bis 15 Gew.-%, besonders bevorzugt von 5 bis 10 Gew.-% - jeweils bezogen auf das Gesamtgewicht der Zubereitung - gewählt.

Es war insbesondere überraschend, dass erfindungsgemäße Zubereitungen mit einem Gehalt an Moisturizern (eine oder mehrere Verbindungen) eine sehr gute Hautbefeuchtungsleistung zeigen, die insbesondere für ölfreie Zubereitungen erstaunlich hoch ist, wobei sich diese positive Wirkung erstaunlicher Weise bereits nach einmaligem Auftragen zeigt.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, 1,2-Propandiol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, jeweils bezogen auf das Gesamtgewicht der Zubereitung.

Besonders vorteilhaft im Sinne der vorliegenden Erfindung können öllösliche Antioxidantien eingesetzt werden. Bevorzugte Antioxidantien sind Vitamin E und dessen Derivate sowie Vitamin A und dessen Derivate.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist dem Fachmann natürlich bekannt, dass kosmetische Zubereitungen zumeist nicht ohne die üblichen Hilfs- und Zusatzstoffe denkbar sind. Die erfindungsgemäßen kosmetischen Zubereitungen können dementsprechend - je nach gewünschtem Anwendungszweck - ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Salze, antimikrobiell, proteolytisch oder keratolytisch wirksame Substanzen oder andere übliche Bestandteile einer kosmetischen Formulierung wie Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen. Auch auf organischen Anionen basierende Elektrolyte sind vorteilhaft, z. B. Lactate, Acetate, Benzoate, Citrate, Aminosäuren, Ethylendiamintetraessigsäure und deren Salze und andere mehr. Als Kationen der Salze werden bevorzugt Ammonium,- Alkalimetall-, Erdalkalimetall,- Magnesium-, Eisen- bzw. Zinkionen verwendet. Es bedarf an sich keiner Erwähnung, dass in Kosmetika nur physiologisch unbedenkliche Elektrolyte verwendet werden sollten. Besonders bevorzugt sind Kochsalz, Magnesiumsulfat, Magnesiumchlorid, Zinksulfat und Mischungen daraus.

Vorteilhaft enthalten die Zubereitungen im Sinne der vorliegenden Erfindung ferner einen oder mehrere Wirkstoffe gewählt aus der Gruppe: Ceramid 3, Traubenkernöl, Biotin, Panthenol, Aloe Vera, Hammamelis-Extrakt, Ginkgo-Extrakt, Honig, Weizenkeimöl und Mandelöl.

Die erfindungsgemäßen Gele können als Grundlage für kosmetische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Besonders bevorzugt im Sinne der vorliegenden Erfindung sind Zubereitungen zur (großflächigen) Anwendung im Körperpflegebereich.

Günstig im Sinne der vorliegenden Erfindung sind auch Zubereitungen, die in der Form eines Sonnenschutzmittels vorliegen. Es ist aber auch vorteilhaft im Sinne der vorliegenden Erfindungen, solche Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet. Vorzugsweise enthalten solche Zubereitungen im Sinne der vorliegenden Erfindung mindestens eine UV-A-Filtersubstanz und/oder mindestens eine UV-B-Filtersubstanz und/oder mindestens ein anorganisches Pigment, zumal UV-Schutzsubstanzen, ebenso wie Antioxidantien und - gewünschtenfalls - Konservierungsstoffe, einen wirksamen Schutz der Zubereitungen selbst gegen Verderb darstellen.

Es ist vorteilhaft im Sinne der vorliegenden Erfindung, wenn die verwendeten UV-Filtersubstanzen wasserlöslich sind.

Erfindungsgemäß vorteilhafte wasserlösliche UV-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Die Gesamtmenge der Filtersubstanzen wird aus dem Bereich von 0,1 bis 30 Gew.-%, vorzugsweise 0,5 bis 10 Gew.-%, insbesondere 1,0 bis 6,0 Gew.-% -jeweils bezogen auf das Gesamtgewicht der Zubereitungen - gewählt, um kosmetische Zubereitungen zur Verfügung zu stellen, die das Haar bzw. die Haut vor dem gesamten Bereich der ultravioletten Strahlung schützen.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| Beispiel | 1 | 2 | 3 | 4 | 5 | 6 | 7 | 8 |
|---|---|---|---|---|---|---|---|---|
| Natriumcarboxymethylstärke | 0,5 | 5,0 | 2,0 | 3,0 | 2,5 | 4,0 | 1,0 | 2,5 |
| Natrium Polyacrylate | 0,25 | 0,1 | 0,5 | | 1,25 | | 0,25 | 0,5 |
| Ammoniumacryloyldimethyltaurat / Vinypyrrolidoncopolymer | 0,25 | 0,2 | 0,5 | 1,5 | | 2,0 | 0,25 | 0,5 |
| Phenylbenzimidazol sulfonic acid | 0,5 | | 1 | | 2 | | 4 | |
| Trisodium EDTA | 0,2 | 0,1 | | 0,05 | | 0,1 | 0,3 | |
| Iminodisuccinat | 0,1 | | 0,1 | | 0,3 | | | 0,5 |
| Phenoxyethanol | 0,3 | 0,1 | | 0,5 | 0,8 | | | 0,4 |
| Parabene | 0,6 | | 0,4 | | 0,4 | | 0,2 | |
| Hexamidin Diisethionat | | 0,1 | | | 0,05 | | 0,1 | |
| Imidodiazolydynl Urea | | | | | | 0,2 | | 0,2 |
| DMDM Hydantoin | | | 0,2 | | | 0,1 | | |
| lodopropynyl Butylcarbamat | | | | 0,2 | | | 0,05 | |
| Glycerin | 10,0 | 3,0 | 7,0 | 8,0 | 15,0 | 20,0 | 0,5 | 2,0 |
| Harnstoff | | 2,0 | | | | | 5,0 | 2,0 |
| Alcohol Denat. | 5,0 | | 2,5 | | | 7,5 | | 7,5 |
| Wasser | ad 100 | ad 100 | ad 100 | ad 100 | ad 100 | ad 100 | ad 100 | ad 100 |

## Patentansprüche

1. Kosmetische Zubereitung in Form eines transparenten bzw. transluzenten Gels, **dadurch gekennzeichnet, dass** sie
i. Wasser,
ii. Natriumcarboxymethylstärke und
iii. ein oder mehrere Hydrokolloide enthält.

2. Zubereitung nach Anspruch 1, **dadurch gekennzeichnet, dass** die Menge an Natriumcarboxymethylstärke aus dem Bereich von 0,5 bis 5 Gew.-% - bezogen auf das Gesamtgewicht der Zubereitung - gewählt wird.

3. Zubereitung nach Anspruch 1, **dadurch gekennzeichnet, dass** die Menge an Natriumcarboxymethylstärke aus dem Bereich von 2 bis 3 Gew.-% - bezogen auf das Gesamtgewicht der Zubereitung - gewählt wird.

4. Zubereitung nach einem der vorhergehenden Ansprüche, **dadurch gekennzeichnet, dass** als Hydrokolloid ein Ammoniumacryloyldimethyltaurate/Vinylpyrrolidoncopolymer gewählt wird.

5. Zubereitung nach einem der vorhergehenden Ansprüche, **dadurch gekennzeichnet, dass** als Hydrokolloid ein Sodium Polyacrylat gewählt wird.

6. Zubereitung nach einem der vorhergehenden Ansprüche, **dadurch gekennzeichnet, dass** das Verhältnis von Natriumcarboxymethylstärke zu Hydrokolloid (insbesondere Ammoniumacryloyldimethyltaurate/Vinylpyrrolidoncopolymere und/oder Sodium Polyacrylate) wie 5 : 1 bis 1 : 1 gewählt wird.

7. Zubereitung nach Anspruch 6, **dadurch gekennzeichnet, dass** das Verhältnis wie 2 : 1 gewählt wird.

8. Zubereitung nach einem der vorhergehenden Ansprüche, **dadurch gekennzeichnet, dass** sie ferner einen oder mehrere Moisturizer enthält.

9. Zubereitung nach Anspruch 8, **dadurch gekennzeichnet, dass** das die Menge an dem oder den Moisturizern aus dem Bereich von 1 bis 20 Gew.-% - bezogen auf das Gesamtgewicht der Zubereitung - gewählt wird.

10. Zubereitung nach Anspruch 8, **dadurch gekennzeichnet, dass** das die Menge an dem oder den Moisturizern aus dem Bereich von 3 bis 15 Gew.-% - bezogen auf das Gesamtgewicht der Zubereitung - gewählt wird.

11. Zubereitung nach Anspruch 8, **dadurch gekennzeichnet, dass** das die Menge an dem oder den Moisturizern aus dem Bereich von 5 bis 10 Gew.-% - bezogen auf das Gesamtgewicht der Zubereitung - gewählt wird.

12. Zubereitung nach Anspruch 8, **dadurch gekennzeichnet, dass** der oder die Moisturizer aus der Gruppe Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff gewählt wird/werden.
